# EUROPÄISCHE PATENTANMELDUNG

(11) **EP 0 550 871 A2**
(43) Veröffentlichungstag der Anmeldung: **14.07.1993**
(21) Anmeldenummer: 92121740.2
(22) Anmeldetag: 21.12.1992
(51) Int. Cl.: C07C 35/08, C07C 29/04

(54) **Verfahren zur Herstellung von Cycloalkanolen**

(30) Priorität: 10.01.1992 DE 4200413
(71) Anmelder: BASF Aktiengesellschaft, D-67063 Ludwigshafen (DE)
(72) Erfinder: Fischer, Rolf, Dr., W-6900 Heidelberg (DE); Pinkos, Rolf, Dr., W-6702 Bad Duerkheim (DE)

(57) **Zusammenfassung**

Verfahren zur Herstellung von Cycloalkanolen durch Umsetzen von Cycloalkenen mit Wasser unter Mitverwendung von Carbonsäuren in Gegenwart von festen sauren Katalysatoren bei einer Temperatur von 60 bis 240°C in flüssiger Phase, dadurch gekennzeichnet, daß man aromatische Carbonsäuren mitverwendet.

## Beschreibung

Die Erfindung betrifft ein Verfahren zur Herstellung von Cycloalkanolen durch Umsetzen von Cycloolefinen mit Wasser in Gegenwart von sauren Katalysatoren.

In der DE-PS 34 41 072 wird ein Verfahren zur Herstellung von Cycloalkanolen beschrieben, bei dem man Cycloolefine mit Wasser in flüssiger Phase bei einer Temperatur von 50 bis 250°C in Gegenwart von Zeolithen mit einem Verhältnis von sauren Stellen an der äußeren Oberfläche zur Gesamtzahl von sauren Stellen ≧ 0,07 umsetzt. Das Verfahren hat den Nachteil, daß der Cycloolefinumsatz gering ist. Bei einer technischen Realisierung müßte ein großer Mengenstrom unumgesetztes Cycloolefin, energetisch aufwendig, vom Cycloalkanol getrennt und zurück gefahren werden.

Nach einem anderen in der EP-A 162 475 beschriebenen Verfahren wird Cycloalkanol durch Umsetzung von Cycloalkenen mit Wasser in Gegenwart von Zeolithen und Säuren wie Broenstedtsäuren, Lewissäuren, anorganischen und organischen Säuren beschrieben. Hierbei lädt der erzielbare Cyclohexenumsatz nicht zu einer Übertragung in den technischen Maßstab ein.

Ferner ist aus der EP-A 341 163 bekannt, daß man Cycloolefine mit Wasser in Gegenwart von Zeolithen bei einer Temperatur von 90 bis 120°C in flüssiger Phase umsetzt, wobei man zusätzlich Carbonsäuren mitverwendet. Bei der Verwendung von Essigsäure bilden sich hierbei bis zu 65 % der entsprechende Ester.

Es war deshalb die technische Aufgabe gestellt, ein Verfahren zur Herstellung von Cycloalkanolen durch Hydratisieren von Cycloalkenen zur Verfügung zu stellen, das mit hohem Umsatz verläuft, eine hohe Selektivität zu Cyclohexanolen ergibt und bei dem möglichst wenig Nebenprodukte wie Ether oder Ester anfallen.

Diese Aufgabe wird gelöst in einem Verfahren zur Herstellung von Cycloalkanolen durch Umsetzung von Cycloalkenen mit Wasser unter Mitverwendung von Carbonsäuren in Gegenwart von festen sauren Katalysatoren bei einer Temperatur von 60 bis 240°C in flüssiger Phase, wobei man aromatische Carbonsäuren verwendet.

Das neue Verfahren hat den Vorteil, daß es hohe Umsätze an Cycloalkenen mit hoher Selektivität zu Cycloalkanolen erlaubt und daß hierbei überraschend wenig Nebenprodukte wie Ether und Ester anfallen. Ferner hat das neue Verfahren den Vorteil, daß die mitverwendeten Carbonsäuren leicht abtrennbar sind.

Es war nicht vorauszusehen, daß aromatische Carbonsäuren, im Gegensatz zu aliphatischen Carbonsäuren, praktisch nicht zu Cyclohexylestern umgesetzt werden (siehe Vergleichsbei-spiel 2).

Bevorzugte Cycloolefine haben 5 bis 8 Kohlenstoffe im Ring und eine olefinische Doppelbindung. Geeignete Cycloolefine sind beispielsweise Cyclopenten, Cyclohexen, Cycloocten. Besondere Bedeutung hat Cyclohexen als Ausgangsstoff erlangt. Es ist auch möglich, Gemische aus Cyclohexen, Cyclohexan und Benzol, wie sie bei der partiellen Hydrierung vom Benzol anfallen zu verwenden und durch Hydratisieren Cyclohexen unter Bildung von Cyclohexanol abzutrennen.

Die Cycloolefine werden mit Wasser umgesetzt. Vorteilhaft verwendet man je Mol Cycloolefin 100 bis 0,04 Mol Wasser. Besonders bewährt hat es sich, wenn man je Mol Cycloolefin 50 bis 0,1, insbesondere 10 bis 1 Mol Wasser anwendet. Besonders vorteilhaft ist es, wenn man Wasser im Überschuß anwendet.

Die Umsetzung wird bei einer Temperatur von 60 bis 240°C durchgeführt. Vorteilhaft hält man eine Temperatur von 70 bis 190°C, insbesondere von 80 bis 160°C ein. Ferner hält man bei der Umsetzung in der Regel einen Druck von 1 bis 100 bar, insbesondere von 1 bis 50 bar ein. Die Druck- und Temperaturbedingungen werden so aufeinander abgestimmt, daß die Ausgangsstoffe und Reaktionsprodukte in flüssiger Phase vorliegen.

Die Umsetzung wird in Gegenwart von festen sauren Katalysatoren durchgeführt. Geeignete feste saure Katalysatoren sind beispielsweise stark-saure Ionenaustauscher wie Sulfonsäuregruppen enthaltendes vernetztes Polystyrol. Eine andere Gruppe von festen sauren Katalysatoren sind Heteropolysäuren und saure praktisch wasserunlösliche Oxide wie Zirkondioxid, Zinndioxid oder Titandioxid, die gegebenenfalls mit zusätzlichen sauren Gruppen wie SO₄²⁻ dotiert sein können. Weiterhin sind geeignete Katalysatoren Zeolithe in der H-Form. Besonders geeignete Zeolithe sind solche aus der Mordenit-Gruppe oder engporige Zeolithe wie die X-, Y- oder L-Zeolithe, z.B. Mordenit, Erionit-, Chabasit- oder Faujasit. Ferner sind ultrastabile Zeolithe aus der Faujasitgruppe, die dealuminiert sind, geeignet.

Besonders vorteilhaft sind Zeolithe mit Pentasilstruktur wie ZSM-5, ZSM-11 und ZBM-10. Diese Zeolithe haben als Grundbaustein einen aus SiO₂-Tetraedern aufgebauten Fünfring gemeinsam, weisen ein hohes Verhältnis von Siliciumdioxid zu Aluminiumoxid auf und haben Porengrößen, die zwischen denen der Zeolithe vom Typ A und denen vom Typ X oder Y liegen.

Je Gewichtsteil cyclisches Olefin wendet man in der Regel 100 bis 0,01 Gew.-Teile feste stark saure Katalysatoren an. Vorteilhaft wendet man je Gewichtsteil cyclische Olefine 10 bis 0,05, insbesondere 7 bis 0,1 Gew.-Teile feste saure Katalysatoren an.

Erfindungsgemäß wird das Verfahren unter Mitverwendung von aromatischen Carbonsäuren durchgeführt. Solche aromatischen Carbonsäuren können bis zu 4 Carboxylgruppen enthalten und zusätzlich unter Reaktionsbedingungen inerte Substituenten wie Alkylgruppen, z.B. mit 1 bis 4 Kohlenstoffatomen Nitrilgruppen, Nitrogruppen oder Halogenatome als Substituenten haben. Vorteilhaft verwendet man aromatische Carbonsäuren die sich von Benzol, Naphthalin, Anthracen oder Phenanthren ableiten. Besonders bevorzugt sind aromatische Carbonsäuren, die sich vom Benzol oder Naphthalin ableiten und 1 oder 2 Carboxylgruppen enthalten. Die bevorzugten Carbonsäuren können auch bis zu 2, unter Reaktionsbedingungen inerte Substituenten haben, wie Alkylgruppen mit 1 bis 4 Kohlenstoffatomen, Nitril- oder Nitrogruppen oder Chloratome. Geeignete aromatische Carbonsäuren sind beispielsweise Terephthalsäure, Isophthalsäure, Phthalsäure, Toluylsäure, Benzoesäure, 3- oder 4-Methylbenzoesäure, 2- oder 4-Chlorbenzoesäure, 1-Naphthoesäure, 1,4-Naphthalindicarbonsäure oder 1,7-Naphthalindicarbonsäure, sowie Anthracen- oder Phenanthrencarbonsäuren.

Vorteilhaft wendet man je Mol cyclisches Olefin 30 bis 0,01 Mol aromatische Carbonsäuren, vorzugsweise von 20 bis 0,05, insbesondere 10 bis 0,1 Mol aromatische Carbonsäuren an.

Das Verfahren nach der Erfindung führt man beispielsweise durch, indem man eine Mischung aus Wasser, Cycloolefin und aromatischer Carbonsäure, in Gegenwart eines suspendierten, festen sauren Katalysators umsetzt und den suspendierten Katalysator abtrennt, oder das Gemisch über einen fest angeordneten festen sauren Katalysator leitet. Aus dem erhaltenen Reaktionsgemisch trennt man Cycloalkanol und nichtumgesetzten Cycloolefin nach bekannten Trennverfahren, z.B. durch Phasentrennung ab. Die verbleibende wäßrige Lösung oder Suspension von aromatischen Carbonsäuren kann wieder zurückgeführt werden. Das Cycloalkanol läßt sich z. B. durch Destillation vom unumgesetzten Cycloolefin trennen.

Cycloalkanole, die nach dem Verfahren der Erfindung hergestellt werden, z.B. Cyclohexanol, sind wertvolle Ausgangsstoffe zur Herstellung von Faserrohstoffen.

Das Verfahren nach der Erfindung sei an folgenden Beispielen veranschaulicht:

### Beispiel 1

In einem 25 ml Druckgefäß wurden 0,9 g Cyclohexen, 1,8 g Wasser, 2,0 g Benzoesäure und 0,6 g eines ZSM-11-Zeolithen in der H-Form unter Rühren 3 h auf 120°C erhitzt. Danach wurde der Reaktionsaustrag für die gaschromatographische Analyse der Reaktionsprodukte mit Aceton verdünnt. Es fanden sich neben unumgesetztem Cyclohexen 43 Mol-% Cyclohexanol.

Als einziges Nebenprodukt bildete sich der Benzoesäurecyclohexylester mit 0,4 Mol-%.

### Beispiel 2

Analog Beispiel 1 wurden 0,9 g Cyclohexen, 1,8 g Wasser, 0,5 g Benzoesäure und 0,6 g eines ZSM-11-Zeoliths in der H-Form 2 h auf 120°C erhitzt. Nach GC-Analyse bildeten sich 27 Mol-% Cyclohexanol. Als einziges Nebenprodukt entstand Benzoesäurecyclohexylester mit 0,02 mol.-%.

### Beispiel 3

Analog Beispiel 2 wurden 0,9 g Cyclohexen, 1,8 g Wasser, 2 g Phthalsäure und 0,6 g eines ZSM-11-Zeoliths in der H-Form 2 h auf 120°C erhitzt. Nach GC-Analyse bildeten sich 35 Mol-% Cyclohexanol bei 98 % Selektivität.

### Beispiel 4

Analog Beispiel 1 wurden 1,0 g Cyclohexen, 1,8 g Wasser, 2 g α-Naphthoesäure und 1 g Ionenaustauscher (Amberlyst XN 1010) 2 h auf 130°C erhitzt. Nach GC-Analyse bildeten sich 14 Mol-% Cyclohexanol. Nebenprodukte wurden nicht gefunden.

### Vergleichsbeispiel 1

Analog Beispiel 1 wurden mit dem gleichen Katalysator und ohne Benzoesäurezusatz 13,5 Mol-% Cyclohexanol (Selektivität 99,5 %) erhalten.

### Vergleichsbeispiel 2

Analog Beispiel 1 wurden 0,82 g Cyclohexen, 0,82 g Wasser, 6 g Essigsäure und 0,55 g eines ZSM-11-Zeoliths in der H-Form 2 h auf 120°C erhitzt. Nach GC-Analyse bildeten sich 15,5 Mol-% Cyclohexanol (Selektivität 20 %) und 61,5 mol.-% Cyclohexylacetat.

## Patentansprüche

1. Verfahren zur Herstellung von Cycloalkanolen durch Umsetzen von Cycloalkenen mit Wasser unter Mitverwendung von Carbonsäuren in Gegenwart von festen sauren Katalysatoren bei einer Temperatur von 60 bis 240°C in flüssiger Phase, dadurch gekennzeichnet, daß man aromatische Carbonsäuren mitverwendet.

2. Verfahren nach Anspruch 1, dadurch gekennzeichnet, daß man Carbonsäuren, die sich vom Benzol, Naphthalin, Anthracen oder Phenantren ableiten, verwendet.

3. Verfahren nach den Ansprüchen 1 und 2, dadurch gekennzeichnet, daß man je Mol Cycloolefin 20 bis 0,05 Mol aromatische Carbonsäuren anwendet.

4. Verfahren nach den Ansprüchen 1 bis 3, dadurch gekennzeichnet, daß man als feste saure Katalysatoren Zeolithe in der H-Form oder stark saure Ionenaustauscher verwendet.

5. Verfahren nach den Ansprüchen 1 bis 4, dadurch gekennzeichnet, daß man je Mol Cycloolefin 50 bis 0,1 Mol Wasser anwendet.

6. Verfahren nach den Ansprüchen 1 bis 5, dadurch gekennzeichnet, daß man eine Temperatur von 80 bis 160°C einhält.

7. Verfahren nach den Ansprüchen 1 bis 6, dadurch gekennzeichnet, daß man einen Druck von 1 bis 50 bar einhält.
